# EUROPEAN PATENT APPLICATION

(11) **EP 4 480 503 A1**
(43) Date of publication of application: **25.12.2024**
(21) Application number: 23796044.8
(22) Date of filing: 07.04.2023
(51) Int. Cl.: A61L 2/10

(54) **UV IRRADIATION DEVICE AND UV IRRADIATION METHOD**

(30) Priority: 27.04.2022 JP 2022073254
(71) Applicant: Yamaguchi University, Yamaguchi 753-8511 (JP); Ushio Denki Kabushiki Kaisha, Tokyo 100-8150 (JP)
(72) Inventor: NISHIKAWA, Jun, Ube-shi Yamaguchi 755-8505 (JP); HARADA, Kouji, Ube-shi Yamaguchi 755-8505 (JP); FUKUDA, Soichiro, Ube-shi Yamaguchi 755-8505 (JP); FUJII, Tomohiro, Ube-shi Yamaguchi 755-8505 (JP); OHASHI, Hiroyuki, Tokyo 100-8150 (JP); KOI, Toru, Tokyo 100-8150 (JP); SUZUKI, Shinji, Tokyo 100-8150 (JP)
(74) Representative: Maiwald GmbH
(86) International application number: PCT/JP2023/014346
(87) International publication number: WO 2023/210298

(57) **Abstract**

There are disclosed an ultraviolet irradiation device and an ultraviolet irradiation method that can easily and appropriately decompose biofilms. The ultraviolet light irradiation device includes a light source part having a light source that emits ultraviolet light in a wavelength range of 190 nm to 240 nm, and a supporting part supporting the light source part to cause the ultraviolet light emitted from the light source to be applied onto a biofilm. The ultraviolet irradiation decomposes the biofilm by irradiating the biofilm with the ultraviolet light.

## Description

### TECHNICAL FIELD

The present invention relates to ultraviolet irradiation devices and methods of ultraviolet irradiation for irradiating biofilms with ultraviolet light.

### BACKGROUND ART

Generally, when organic matter or ions adhere to the surface of a material that is in contact with a nutrient solution such as water, a conditioning film is formed on the surface. When bacteria adhere to the conditioning film, they secrete extracellular polymeric substances (EPSs) to form a biofilm.

Biofilms can exist in various environments, such as soil, riverbeds, plant rhizospheres, bathroom drains, kitchen sink drains, and inside human bodies (e.g., oral cavities).

Biofilms play an important role in carbon and nitrogen cycling in nature and in effluent treatment and decomposition of petroleum pollution in the ocean in industrial fields.

For example, Patent Document 1 (Japanese Utility Model Registration No. 3233968) discloses a biofilm treatment system that uses biofilms to remove organic and inorganic substances from surrounding liquids. Patent document 1 (Japanese Utility Model Registration No. 3233968) discloses that the biofilm treatment system can use a UVC device that uses glass that transmits ultraviolet rays with a wavelength of 207 nm to 222 nm, which can kill bacteria and other microbes without adversely affecting human skin and eyes. However, there is no specific disclosure of how the UVC device is applied to biofilm treatment.

### BACKGROUND DOCUMENT

### Patent Document

Patent Document 1: Japanese Utility Model Registration No. 3233968

### SUMMARY OF THE INVENTION

### PROBLEM TO BE SOLVED BY THE INVENTION

Whereas biofilms play an important role in nature and industry as described above, they are known to cause problems such as metal corrosion, drainage pipe contamination, and biofouling of marine structures. In particular, in the biological field, dental plaque is a typical example of a biofilm formed in an oral cavity, which causes periodontal disease to progress. In the medical field, biofilms are considered to cause bacterial contamination of medical devices. Furthermore, many bacterial infections are caused by biofilms, and treatment thereof is extremely difficult.

Usually, multiple species of microorganisms, including bacteria, live in biofilms, and some of them may be harmful to human bodies. Accordingly, it is desirable to decompose and/or remove biofilms formed in spaces related to people's living and formed in human bodies, and/or to sterilize harmful microorganisms contained in biofilms in order to control their adverse effects on human bodies.

Conventionally, the use of agents such as antimicrobial agents has been attempted to decompose and/or remove biofilms. However, biofilms are resistant to stresses such as heat and dryness, as well as to agents such as antibiotics. Therefore, it is difficult to decompose and/or remove biofilms with agents, and it is also difficult to kill harmful bacteria contained in biofilms.

Accordingly, it is believed that it is effective to physically (or surgically in cases of human bodies) remove biofilms. However, physical removal methods are not always able to completely remove biofilms.

Accordingly, it is an object of the present invention to provide an ultraviolet irradiation device and an ultraviolet irradiation method that can easily and appropriately decompose biofilms.

### MEANS FOR SOLVING THE PROBLEM

In order to solve the above problem, according to an aspect of the present invention, there is provided an ultraviolet irradiation device including a light source part having a light source that emits ultraviolet light in a wavelength range of 190 nm to 240 nm, and a supporting part supporting the light source part to cause the ultraviolet light emitted from the light source to be applied onto a biofilm for decomposing the biofilm.

The inventors have newly discovered that biofilms can be decomposed by irradiating them with ultraviolet light in a wavelength range of 190 nm to 240 nm. Here, "decomposition of biofilm" includes decomposition of extracellular polymer substances (EPSs) constituting the biofilm to destroy the biofilm structure.

As described above, by adopting a configuration in which the light source part is supported by a supporting part to irradiate the biofilm with ultraviolet light in the wavelength range of 190 nm to 240 nm, the biofilm can be irradiated with the ultraviolet light appropriately and can be easily decomposed. Therefore, various problems caused by biofilms can be avoided. In addition, since the ultraviolet light in the wavelength range of 190 nm to 240 nm has little adverse effect on human and animal cells, it is possible to irradiate biofilms formed in spaces in which there may be humans and/or animals and in the bodies of humans and animals with the ultraviolet light to decompose the biofilms.

The ultraviolet irradiation device may inactivate the bacteria that have formed the biofilm, and microorganisms and/or viruses contained in the biofilm, by irradiating the biofilm with the ultraviolet light.

In this way, the bacteria that have formed the biofilm can be sterilized to stop the increase in biofilm production. In addition, by inactivating the microorganisms and/or viruses contained in the biofilm, the adverse effects of the microorganisms and/or viruses on the human body can be minimized.

Furthermore, the ultraviolet irradiation device may irradiate the biofilm formed on either a surface of an oral cavity or an inner lining of a large intestine of a human with the ultraviolet light. In this case, the progression of periodontal disease, the development of colorectal cancer, and the occurrence of infectious diseases can be reduced.

Furthermore, in the ultraviolet irradiation device, the light source part may further have an optical filter that transmits ultraviolet light in a wavelength range of 190 nm and 240 nm and blocks transmission of UV-C waves having longer wavelengths than 240 nm, so that the ultraviolet irradiation device may radiate the ultraviolet light in the wavelength range of 190 nm to 240 nm that passes through the optical filter among the ultraviolet light emitted from the light source.

In this case, only light in the wavelength range with minimal adverse effects on the human body and animals can be emitted from the light source part.

According to another aspect of the present invention, there is provided a method of ultraviolet irradiation including a step of emitting ultraviolet light in a wavelength range of 190 nm to 240 nm from a light source; and a step of irradiating a biofilm formed on either a surface of an oral cavity or an inner lining of a large intestine of a human with the ultraviolet light emitted from the light source to decompose the biofilm.

By irradiating the biofilm with ultraviolet light in the wavelength range of 190 nm to 240 nm, the biofilm can be easily and appropriately decomposed. In addition, since the ultraviolet light in the wavelength range of 190 nm to 240 nm has little adverse effect on human and animal cells, it is possible to irradiate biofilms formed in the bodies of humans and animals with the ultraviolet light to decompose the biofilms.

The method of ultraviolet irradiation may further include a step of irradiating the biofilm with the ultraviolet light emitted from the light source to inactivate bacteria that have formed the biofilm, and microorganisms and/or viruses contained in the biofilm.

In this way, the bacteria that have formed the biofilm can be sterilized to stop the increase in biofilm production. In addition, by inactivating the microorganisms and/or viruses contained in the biofilm, the adverse effects of the microorganisms and/or viruses on the human body can be minimized.

The method of ultraviolet irradiation may further include a step of stopping emission of the ultraviolet light after emitting the ultraviolet light; and a step of administering an agent to the biofilm.

By irradiating the biofilm with ultraviolet light in the wavelength range of 190 nm to 240 nm, the cell membrane of the bacterial cells contained in the biofilm can be destroyed and the agent can easily penetrate into the bacteria. By administering the drug after ultraviolet irradiation, the bacteria in the biofilm can be more appropriately sterilized. In addition, by administering the drug after the ultraviolet radiation is stopped, it is possible to prevent the drug from being decomposed by the ultraviolet light.

### EFFECTS OF THE INVENTION

In accordance with the present invention, by irradiating biofilms with ultraviolet light with a wavelength of 190 nm to 240 nm, the biofilms can be easily and appropriately decomposed.

Objects, aspects, and effects of the present invention described above, as well as objects, aspects, and effects of the present invention not described above, will be understood by those skilled in the art from the following detailed description of the invention with reference to the accompanying drawings and claims.

### BRIEF DESCRIPTION OF THE DRAWINGS

FIG. 1 is a chart showing an ultraviolet absorption spectrum of proteins;
FIG. 2 is a schematic diagram showing an experimental system for verification experiments;
FIG. 3 shows results of crystal violet staining of biofilms after irradiation with far ultraviolet light;
FIG. 4 shows results of crystal violet staining of biofilms after irradiation with ultraviolet light;
FIG. 5 is a graph showing amounts of biofilm formation after irradiation with far ultraviolet light;
FIG. 6 is a graph showing amounts of biofilm formation after irradiation with ultraviolet light;
FIG. 7 shows MTS test results after irradiation with far ultraviolet light;
FIG. 8 shows MTS test results after being irradiated with ultraviolet light;
FIG. 9 is a graph showing amounts of viable bacteria after irradiation with far ultraviolet light;
FIG. 10 is a graph showing amounts of viable bacteria after irradiation with ultraviolet light;
FIG. 11 shows results of SYTO9 staining and PI staining;
FIG. 12 is a schematic diagram showing an example of an ultraviolet irradiation device; and
FIG. 13 is a schematic diagram showing another example of an ultraviolet irradiation device.

### DESCRIPTION OF EMBODIMENT

Hereinafter, with reference to the accompanying drawings, an embodiment of the present invention will be described.

The following is a description of an embodiment of an ultraviolet irradiation device that irradiates a biofilm with ultraviolet light to achieve decomposition (destruction) of biofilms and/or inactivation of microorganisms, viruses, etc. contained in the biofilms.

The term "decomposition of biofilms" in the embodiment includes decomposing extracellular polymeric substances (EPSs) that constitute biofilms and destroying the structures of the biofilms. In addition, "inactivation" in the embodiment refers to killing microorganisms and viruses (or causing them to lose their infectivity and/or toxicity).

The inventors have newly discovered that biofilms can be decomposed by irradiation with ultraviolet light with a wavelength of 190 nm to 240 nm.

The reason for which biofilms can be decomposed by irradiation with ultraviolet light in the above wavelength range is not exactly clear, but is thought to be as follows.

EPSs, which form the matrix of biofilms, include polysaccharides (exopolysaccharides), proteins, lipids, extracellular DNA, etc.

Let us consider ultraviolet properties for proteins.

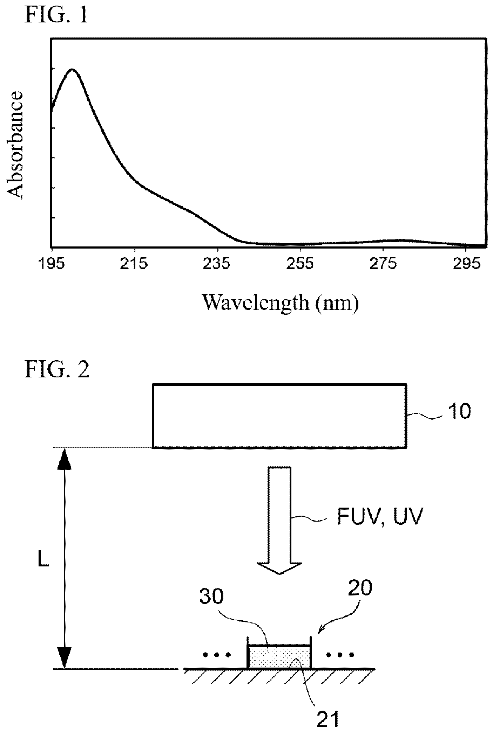

FIG. 1 is a chart showing an ultraviolet absorption spectrum of proteins. As shown in FIG. 1, proteins have an absorption peak at a wavelength of 200 nm, but do not absorb much ultraviolet light with wavelengths of 240 nm or more.

Accordingly, one reason for which biofilms can be decomposed by irradiation with ultraviolet light in the wavelength range of 190 nm to 240 nm may be thought to be that fundamental components of biofilms are proteins, and ultraviolet light in the wavelength range of 190 nm to 240 nm is well absorbed by proteins.

In addition, sugars, proteins, lipids, and DNA in EPSs have carbon (C) double bonds. The photon energy of ultraviolet light is higher at shorter wavelengths; for example, ultraviolet light at 222 nm can cleave carbon double bonds.

Accordingly, another reason for which biofilms can be decomposed by irradiation with ultraviolet light in the wavelength range of 190 nm to 240 nm, especially at a wavelength of 222 nm, may be thought to be that molecular chains in biofilms absorb ultraviolet light at the wavelength of 222 nm and are decomposed. The above-described fact that ultraviolet light at the wavelength of 222 nm specifically acts on carbon double bonds and contributes to decomposition of biofilms is a new finding discovered by the inventors.

An ultraviolet irradiation device according to the embodiment irradiates a biofilm with ultraviolet light in a wavelength range of 190 nm to 240 nm (preferably 200 nm to 230 nm) to decompose the biofilm.

The ultraviolet irradiation device according to the embodiment irradiates the biofilm with ultraviolet light in the wavelength range of 190 nm to 240 nm (preferably 200 nm to 230 nm) to inactivate the bacteria that have formed (secreted) the biofilm as well as harmful microorganisms and/or viruses contained in the biofilm at the same time or at the substantially same time of decomposition of the biofilm.

For example, ultraviolet light at a wavelength of 222 nm emitted from a KrCI excimer lamp and ultraviolet light at a wavelength of 207 nm emitted from a KrBr excimer lamp are both suitable for decomposing biofilms and sterilizing and/or inactivating bacteria, etc.

In the following description, ultraviolet rays in the wavelength range of 190 nm to 240 nm will be referred to as "far ultraviolet", "far UV", or "FUV", whereas ultraviolet rays in the wavelength range of 240 nm to 400 nm will be referred to simply as "ultraviolet", or "UV".

The inventors verified the decomposition effect for biofilms and the sterilization effect for bacteria contained in biofilms in cases in which biofilms were irradiated with far ultraviolet (FUV) light in the wavelength range of 190 nm to 240 nm and ultraviolet (UV) light in the wavelength range of 240 nm to 400 nm, respectively. The following is a specific description of the verification experiments and their results.

### Experiment 1

Amounts of biofilm decomposition were quantified in cases in which biofilms were irradiated with far ultraviolet light in the wavelength range of 190 nm to 240 nm and ultraviolet light in the wavelength range of 240 nm to 400 nm, respectively.

The method for quantifying the amounts of biofilm decomposition used measuring amounts of biofilm by the crystal violet (CV) staining method.

FIG. 2 is a schematic diagram showing an experimental system of the verification experiments.

As shown in FIG. 2, biofilms 30 formed on the bottom surfaces 21 of wells 20 of a microplate were irradiated with far ultraviolet (FUV) or ultraviolet (UV) light emitted from an ultraviolet irradiation device 10 that was disposed above the biofilms 30. Then, the amounts of decomposition of the biofilms 30 were quantified.

As the ultraviolet irradiation device 10 that emits far ultraviolet light, "Care222" manufactured by Ushio, Inc. was used. This far ultraviolet irradiation device emits far ultraviolet light in the wavelength range of 190 nm to 240 nm having a peak wavelength of 222 nm.

As the ultraviolet irradiation device 10 that emits ultraviolet light in the wavelength range of 240 nm to 400 nm, "SLUV-4" manufactured by Az One Corporation was used. This ultraviolet irradiation device is switchable between ultraviolet radiation at a wavelength of 254 nm and ultraviolet radiation at a wavelength of 365 nm. In Experiment 1, ultraviolet radiation of 254 nm wavelength was selected.

Each of the ultraviolet irradiation devices 10 was placed such that the light-emitting surface of the ultraviolet irradiation device 10 faced the surfaces of the biofilms 30 and was separated from the bottom surfaces 21 of the wells 20 by a distance L upward. The distance L may be arbitrary.

In Experiment 1, following processes (1) through (4) were performed in order.

### (1) Biofilm Formation

Biofilms 30 were formed in the wells 20 of the microplate. Each biofilm 30 contained as the matrix EPSs secreted by Fusobacterium nucleatum, which is a periodontal disease bacterium. Biofilm formation was performed by the following procedure.

First, frozen liquid containing Fusobacterium nucleatum was poured onto a GAM agar medium, and the GAM agar medium was placed in an airtight container (square jar) together with an anaerobic culture agent, "Anaeropack" manufactured by Mitsubishi Gas Chemical Company, Inc.. Then, Fusobacterium nucleatum in the GAM agar medium was incubated anaerobically for 72 hours under a constant condition at 37 degrees Celsius.

Next, the culture medium was adjusted to 107 CFU/ml with a phosphate buffered saline (PBS) and further diluted to 106 CFU/ml with a GAM liquid medium.

The bacterial solution was mixed well, and 100 µl of the solution was inoculated into each well 20 of a 96-well microplate. Then, the Fusobacterium nucleatum in the wells was incubated anaerobically for 72 hours under a constant condition at 37 degrees Celsius, so that a biofilm 30 was formed on the bottom surface 21 of each of the wells 20.

### (2) Far Ultraviolet And Ultraviolet Irradiation

After the biofilm 30 was formed on the bottom surface 21 of each well 20 in the above-mentioned biofilm formation process, the GAM liquid medium was removed from each well 20.

Each of the wells 20 of the microplate was then washed with 100 µl of a PBS.

Then, the ultraviolet irradiation device 10, "Care222", was used to irradiate the biofilms 30 formed in the wells 20 with far ultraviolet light in a wavelength range of 190 nm to 240 nm having a peak wavelength of 222 nm.

For a comparison purpose, "SLUV-4" was used for the ultraviolet irradiation device 10 to irradiate the biofilms 30 formed in other wells 20 with ultraviolet light at a wavelength of 254 nm.

The irradiation time periods of the far ultraviolet light with the peak wavelength of 222 nm were defined so that the ultraviolet irradiation dose (dose amount) was 0 mJ/cm², 60 mJ/cm², 175 mJ/cm², and 300 mJ/cm².

The irradiation time periods of the ultraviolet light at the wavelength of 254 nm were also defined so that the ultraviolet irradiation dose (dose amount) was 0 mJ/cm², 60 mJ/cm², 175 mJ/cm², and 300 mJ/cm² as in the case of far ultraviolet light.

Thus, biofilms 30 were irradiated with equal dose amounts for the far ultraviolet light and the ultraviolet light.

### (3) Crystal Violet Staining

The biofilms 30 after being irradiated with the far ultraviolet light or the ultraviolet light in the above-mentioned far ultraviolet and ultraviolet irradiation process were stained with crystal violet. Crystal violet is a basic purple dye with a triphenylmethane skeleton and can stain EPSs. Staining with crystal violet was performed by the following procedure.

First, 100 µl of the GAM liquid medium was poured into each of the wells 20 after the ultraviolet or ultraviolet irradiation, and the remaining bacteria in the wells were incubated anaerobically for 24 hours under a constant condition at 37 degrees Celsius. After incubation, the GAM liquid medium was removed. Each of the wells 20 was then washed with 100 µl of the PBS.

Next, 100 µl of crystal violet solution at a concentration of 1 % was added to each of the wells 20, and the biofilms were stained for 30 minutes.

### (4) Light Absorbance Measurement

Light absorbance measurement was performed to quantify the amounts of biofilms. The absorbance measurement procedure was performed by the following procedure.

After staining the biofilms in the above-mentioned crystal violet staining process, each of the wells 20 was washed twice with 100 µl of the PBS. After washing, each of the wells 20 was dried for 10 minutes. Then, ethanol at 95 % concentration was put into each well and left at room temperature for 15 minutes to elute the crystal violet and to solve it in ethanol.

The light absorbance of the solution of crystal violet in ethanol was then measured with a microplate reader. The wavelength of the measurement light was 590 nm.

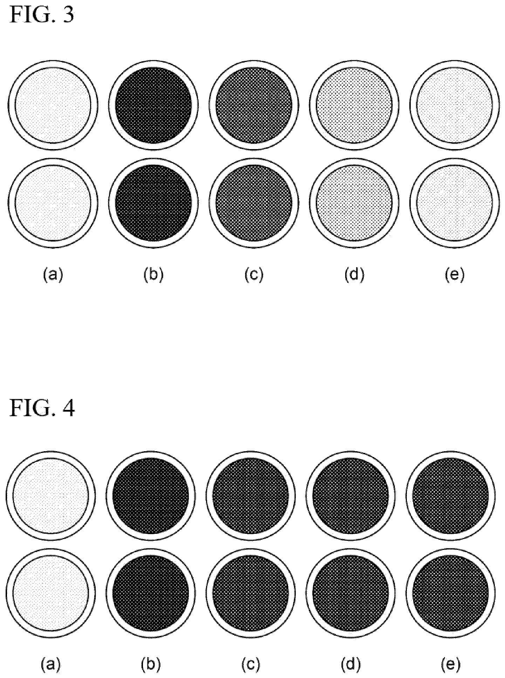

FIG. 3 is a schematic diagram showing results of crystal violet staining of the biofilms after irradiation with far ultraviolet light in the wavelength range of 190 nm to 240 nm having the peak wavelength of 222 nm. FIG. 4 is a schematic diagram showing results of crystal violet staining of biofilms after irradiation with ultraviolet light at the wavelength of 254 nm.

In each of FIGS. 3 and 4, part (a) shows the results of crystal violet staining in a case in which no biofilm was formed (blank), part (b) shows the results of crystal violet staining of the biofilms in a case in which the dose amount was 0 mJ/cm², part (c) shows the results of crystal violet staining of the biofilms in a case in which the dose amount was 60 mJ/cm², part (d) shows the results of crystal violet staining of the biofilms in a case in which the dose amount was 175 mJ/cm², and part (e) shows the results of crystal violet staining of the biofilms in a case in which the dose amount was 300 mJ/cm².

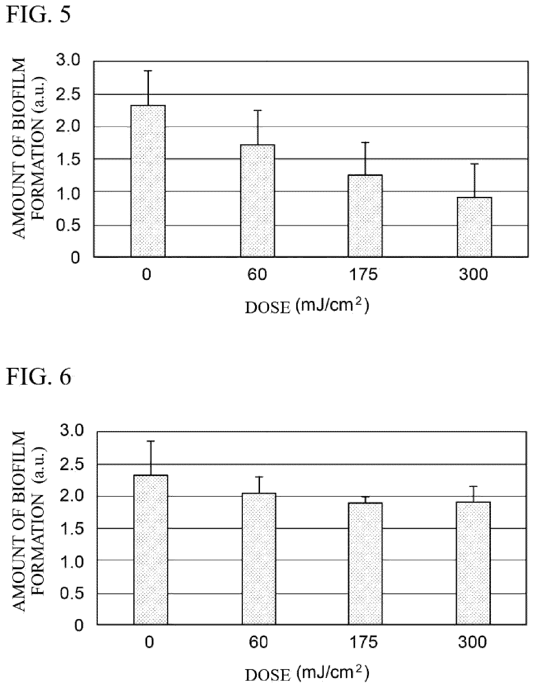

FIG. 5 shows amounts of biofilm formation calculated based on the light absorbances of the stained samples after irradiation with far ultraviolet light in the wavelength range of 190 nm to 240 nm having the peak wavelength of 222 nm. FIG. 6 shows amounts of biofilm formation calculated based on the light absorbances after irradiation with ultraviolet light at the wavelength of 254 nm.

FIGS. 5 and 6 show the amounts of the biofilms in cases in which the dose amount was 0 mJ/cm², 60 mJ/cm², 175 mJ/cm², and 300 mJ/cm².

As shown in FIG. 3, in cases in which the biofilms were irradiated with far ultraviolet light having the peak wavelength of 222 nm, the color of the biofilms became lighter as the irradiation dose (dose amount) increased. This suggests that the amount of biofilm decomposition increases as the irradiation dose of far ultraviolet light increases.

In fact, from the amounts of biofilm formation quantified from the absorbance measurements, as shown in FIG. 5, it was recognized that the amounts of formed biofilms reduced as the irradiation dose of far ultraviolet light increased.

On the other hand, as shown in FIG. 4, in cases in which biofilms were irradiated with ultraviolet light at the wavelength range of 254 nm, the color of the biofilms remained dark and was almost unchanged even as the irradiation dose (dose amount) increased. This suggests that biofilms are hardly decomposed by being irradiated with ultraviolet light at the wavelength of 254 nm.

From the amounts of biofilm formation quantified from the absorbance measurements, as shown in FIG. 6, it was recognized that the amounts of formed biofilms did not change much even as the irradiation dose of ultraviolet light increased.

### Experiment 2

The sterilization effects for bacteria contained in biofilms were quantified in cases in which biofilms were irradiated with far ultraviolet light in the wavelength range of 190 nm to 240 nm and ultraviolet light in the wavelength range of 240 nm to 400 nm, respectively.

The method for quantifying bacterial sterilization used measuring the metabolic activity of viable bacteria in biofilms by the MTS method (MTS assay).

The experimental system was the same as in Experiment 1.

In Experiment 2, the biofilm formation process and the far ultraviolet and ultraviolet irradiation process were performed in the same manner as in Experiment 1. Then, an MTS test was performed using the following procedure to quantify the survival rate of cells in the biofilms.

First, 100 µl of the GAM liquid medium was poured into each of the wells 20 after being irradiated with far ultraviolet light or ultraviolet light, and the remaining bacteria in the wells were incubated anaerobically for 24 hours under a constant condition at 37 degrees Celsius. After incubation, the GAM liquid medium was removed. Each of the wells 20 was then washed with 100 µl of the PBS.

After washing, 100 µl of the PBS was poured into each of the wells 20, and 20 µl of an MTS reagent was added to each of the wells 20. Then, each of the wells 20 was light-shielded and the remaining bacteria in the wells were incubated for three hours under a contact condition at 37 degrees Celsius.

Then, 100 µl of a reaction solution was dispensed into each of the wells 20, and the light absorbance of the sample in each well was measured with the microplate reader. The measurement wavelength was 490 nm.

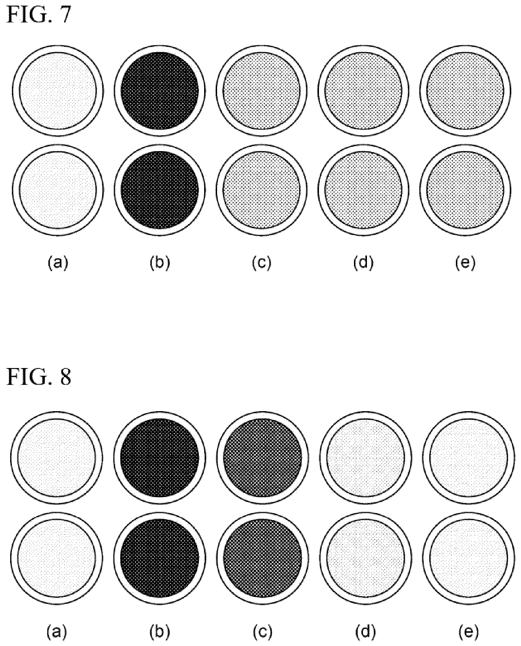

FIG. 7 is a schematic diagram showing the appearances of the samples in the wells 20 after dispensing the reaction solution into each of the wells 20 in the MTS test after irradiation with far ultraviolet light in the wavelength range of 190 nm to 240 nm having the peak wavelength of 222 nm.

FIG. 8 is a schematic diagram showing the appearances of the samples in the wells 20 after dispensing the reaction solution into each of the wells 20 in the MTS test after irradiation with ultraviolet light at the wavelength of 254 nm.

In each of FIGS. 7 and 8, part (a) shows the MTS test results in a case in which no biofilm was formed (blank), part (b) shows the MTS test results for the biofilms in a case in which the dose amount was 0 mJ/cm², part (c) shows the MTS test results for the biofilms in a case in which the dose amount was 60 mJ/cm², part (d) shows the MTS test results for the biofilms in a case in which the dose amount was 175 mJ/cm², and part (e) shows the MTS test results for the biofilms in a case in which the dose amount was 300 mJ/cm².

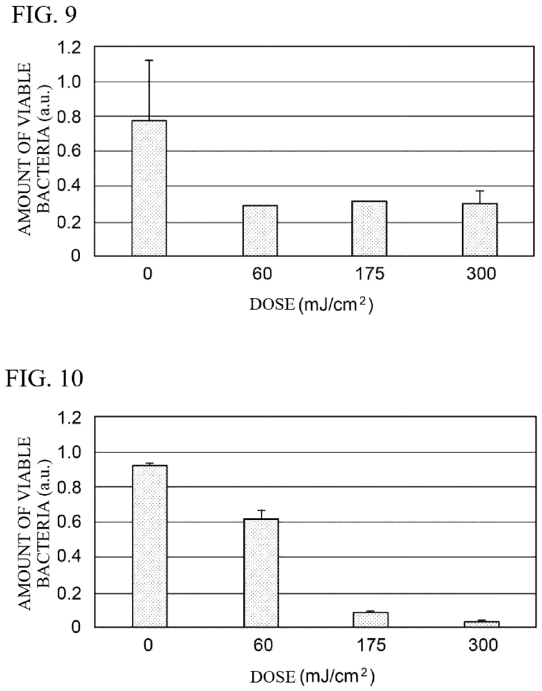

FIG. 9 shows amounts of viable Fusobacterium nucleatum after irradiation with far ultraviolet light in the wavelength range of 190 nm to 240 nm having the peak wavelength of 222 nm. Similarly, FIG. 10 shows amounts of viable Fusobacterium nucleatum after irradiation with ultraviolet light at the wavelength of 254 nm.

FIGS. 9 and 10 show the amounts of viable bacteria in cases in which the dose amount was 0 mJ/cm², 60 mJ/cm², 175 mJ/cm², and 300 mJ/cm².

As shown in parts (c) to (e) in FIG. 7, the color of the samples in the wells 20 became lighter after irradiation with 60 mJ/cm² of far ultraviolet light. This suggests that irradiation with far ultraviolet light disinfects the bacteria in the biofilms.

In fact, from the amounts of viable bacteria quantified from the MTS test, as shown in FIG. 9, it was recognized that the amount of the viable Fusobacterium nucleatum in the biofilms after irradiation with 60 mJ/cm² of far ultraviolet light was less than that before ultraviolet irradiation.

On the other hand, as shown in part (c) in FIG. 8, at the time of irradiation with 60 mJ/cm² of ultraviolet light at the wavelength range of 254 nm, the color of the samples in the wells 20 faded only to a certain extent in comparison with that before ultraviolet irradiation. However, as shown in parts (d) and (e) in FIG. 8, after irradiation with 175 mJ/cm² of ultraviolet light at the wavelength of 254 nm, the color of the samples in the wells 20 became noticeably lighter, and was lighter than the color after irradiation with 175 mJ/cm² of far ultraviolet light shown in part (d) in FIG. 7. Furthermore, the color of the samples in the wells 20 at the time of irradiation with 175 mJ/cm² of ultraviolet light at the wavelength of 254 nm was lighter than the colors of the samples irradiated with 60 mJ/cm² and more of far ultraviolet light shown in FIG. 7.

This suggests that irradiation with 175 mJ/cm² or more of ultraviolet light of which the wavelength is 254 nm can achieve sterilization of Fusobacterium nucleatum better than irradiation with 60 mJ/cm² or more of far ultraviolet light.

From the amounts of viable bacteria quantified from the MTS test, as shown in FIG. 10, it was recognized that the amounts of viable Fusobacterium nucleatum in the biofilms after irradiation with 175 mJ/cm² of ultraviolet light at the wavelength of 254 nm were significantly less than those after irradiation with 60 mJ/cm² of far ultraviolet light.

The results of Experiments 1 and 2 above verified that irradiating biofilms with far ultraviolet light could decompose the biofilms and could sterilize bacteria in the biofilms.

It was also found that irradiating biofilms with ultraviolet light of which the wavelength is 254 nm did not decompose the biofilms, but could sterilize bacteria in the biofilms.

Thus, it was found that far ultraviolet light and ultraviolet light were both capable of sterilizing bacteria in biofilms. The inventors performed Experiment 3, which will be described below, for researching as to whether or not there is any difference in the bactericidal mechanism by far ultraviolet light and ultraviolet light.

### Experiment 3

The inventors researched the sterilization of bacteria contained in biofilms by irradiating the biofilms with far ultraviolet light in the wavelength range of 190 nm to 240 nm and with ultraviolet light in the wavelength range of 240 nm to 400 nm, respectively.

The method for researching bacterial sterilization used introducing staining reagents into biofilms after irradiation with far ultraviolet and ultraviolet light to stain the nucleic acid (DNA) of the cells in the biofilms for researching states of the cells in the biofilms.

The experimental system was the same as in Experiment 1.

Two nuclear staining reagents, SYTO9 and propidium iodide (PI), were used.

SYTO9 staining reagent is a membrane-permeable DNA staining reagent and can stain DNA green, independent of the states of the cell membrane.

PI staining reagent is another DNA staining reagent and is not membrane-permeable. PI staining reagent can stain only the DNA of cells with damaged cell membranes (dead cells) red.

In Experiment 3, the biofilm formation process and the far ultraviolet and ultraviolet irradiation process were performed in the same manner as in Experiment 1. Then, a staining process was performed using the following procedure to research the states of bacteria in the biofilms.

First, 100 µl of the GAM liquid medium was poured into each of the wells 20 after being irradiated with far ultraviolet light or ultraviolet light, and the remaining bacteria in the wells were incubated anaerobically for 24 hours under a constant condition at 37 degrees Celsius. After incubation, the GAM liquid medium was removed. Each of the wells 20 was then washed with 100 µl of the PBS.

After washing, 100 µl of a mixture of SYTO9 staining solution with a concentration of 2.5 µM and PI staining solution with a concentration of 3.0 µM was poured into each of the wells 20. Then, each of the wells 20 was light-shielded and left to stand for 30 minutes.

Each well was then washed with 100 µl of the PBS and observed with a fluorescence microscope.

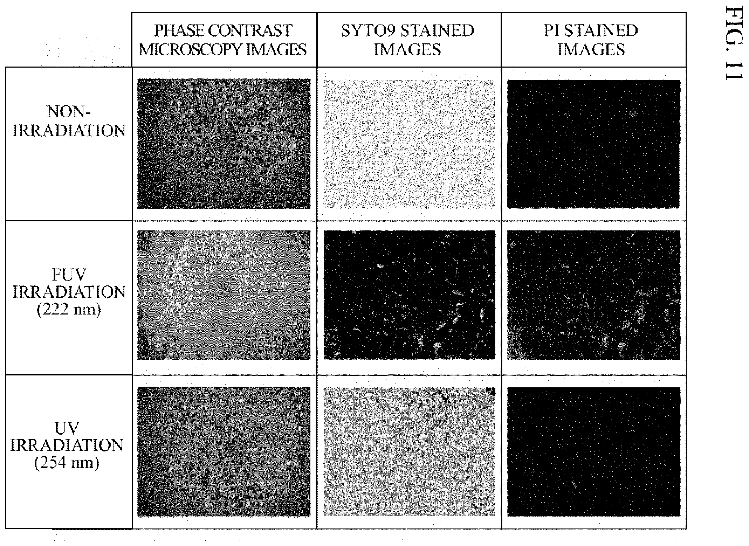

FIG. 11 shows phase contrast microscopy images, SYTO9 stained images, and PI stained images in cases of no irradiation, irradiation with 300 mJ/cm² of far ultraviolet light with the peak wavelength of 222 nm (FUV irradiation), and irradiation with 300 mJ/cm² of ultraviolet light at the wavelength of 254 nm (UV irradiation). In FIG. 11, the gray areas in the SYTO9-stained images are actually green, and the gray areas in the PI-stained images are actually red.

As shown in the top part of FIG. 11, in the case of non-irradiation, in which the biofilm was not irradiated with far-ultraviolet light and ultraviolet light, the SYTO9 stained image was stained green overall, whereas the PI stained image was barely stained red.

The reason for which the SYTO9 stained image was stained green overall in the case of non-irradiation is that the biofilm was still formed, and the bacteria were distributed throughout the biofilm. In other words, the result of SYTO9 staining in Experiment 3 shows generally the area of biofilm formation.

In addition, in the case of non-irradiation, the PI staining showed almost no staining, which suggests that almost all the bacteria in the biofilm were alive.

In contrast, for the biofilm irradiated with 300 mJ/cm² of far ultraviolet light in the wavelength range of 190 nm to 240 nm having the peak wavelength of 222 nm, as shown in the middle part of FIG. 11, the SYTO9 stained image showed sparse staining, and the PI stained image showed that almost the same regions as those stained by SYTO9 staining were stained.

The result of SYTO9 staining confirms that the biofilm was decomposed by far-ultraviolet irradiation and remained only sparsely.

In addition, the PI staining showed that almost the same regions as those stained by SYTO9 staining were stained, which confirms that the bacteria in the biofilm were dead bacteria with damaged cell membranes.

On the other hand, in the biofilm irradiated with 300 mJ/cm² of ultraviolet light at the wavelength of 254 nm, almost the entire area was stained green by SYTO9 staining and almost the entire area was not stained by PI staining, as shown in the bottom part of FIG. 11.

The result of SYTO9 staining confirms that the biofilm was not decomposed by ultraviolet light at the wavelength of 254 nm wavelength.

In addition, since there was almost no staining by PI staining, it could be confirmed that there were almost no dead bacteria with damaged cell membranes in the biofilm.

However, by the MTS test in Experiment 2, it was confirmed that most of the bacteria in the biofilms were dead by irradiation with 300 mJ/cm² of ultraviolet light at the wavelength of 254 nm. Therefore, it can be considered that although the biofilm remained formed without decomposition even after irradiation with ultraviolet light at the wavelength of 254 nm, the bacteria contained in the biofilm were dead. It can be considered that cell death of the bacteria may be caused by a reason other than damaged cell membranes, such as DNA damage.

Bacterial cells have nucleic acids (DNA and RNA), which control genetic information. When exposed to ultraviolet light, nucleic acids absorb the light, and DNA binding is degraded. This leads to disruption of gene transcription and disturbance of metabolism, which causes death. In other words, when exposed to ultraviolet light, bacteria lose their metabolic and proliferating abilities. Cell death due to DNA damage is caused not only by ultraviolet irradiation at the wavelength of 254 nm, but also by far-ultraviolet irradiation at the peak wavelength of 222 nm.

In addition, bacteria and viruses share a common mechanism of inactivation in that DNA binding is damaged by ultraviolet irradiation. Accordingly, if a biofilm contains microorganisms or viruses other than the bacteria that have formed (secreted) the biofilm, ultraviolet irradiation (including far-ultraviolet irradiation) is considered to inactivate the microorganisms and viruses.

Through Experiments 1-3 described above, the inventors were able to verify the following:
(1) Biofilms were decomposed by irradiating with far ultraviolet light in the wavelength range of 190 nm to 240 nm.
(2) Bacteria in the biofilms were sterilized by irradiating with far ultraviolet light in the wavelength range of 190 nm to 240 nm. The bactericidal mechanism is thought to be DNA damage in the bacteria and disruption of the cell membrane. Microorganisms and/or viruses that may be contained in the biofilm were also inactivated.

In addition, as mentioned above, in a case in which the biofilm was irradiated with ultraviolet light at the wavelength of 254 nm, the biofilm was not decomposed, but the bacteria within the biofilm could be sterilized. The reason for this is not exactly clear. However, as mentioned above, EPSs of biofilms are mainly composed of polysaccharides (exopolysaccharides) and proteins, and ultraviolet light at the wavelength of 254 nm has good permeability to proteins as shown in FIG. 1. Accordingly, the reason may be that ultraviolet light at the wavelength of 254 nm could penetrate the biofilm without being absorbed and the bacteria in the biofilm were killed by direct irradiation of ultraviolet light at the wavelength of 254 nm.

Therefore, it is considered that ultraviolet light at the wavelength of 254 nm can efficiently sterilize bacteria in biofilms.

However, even after irradiation with ultraviolet light at the wavelength of 254 nm, the biofilm remains undecomposed, so that bacteria can easily adhere to it again. Once bacteria adhere, the biofilm is reproduced.

In addition, since ultraviolet light at the wavelength of 254 nm penetrates biofilms, it can reach the surface of a substance on which a biofilm is formed. For example, if a biofilm is formed in a human oral cavity, ultraviolet light at the wavelength of 254 nm applied to the biofilm will reach the oral surface. Since oral tissues contain proteins, ultraviolet light at the wavelength of 254 nm that reaches the oral surface can penetrate into the oral tissues and can damage the cells in the tissues. The same is true for ultraviolet light at the wavelength of 240 nm or longer.

On the other hand, ultraviolet rays with a wavelength of approximately 200 nm are absorbed on the surface of human skin (e.g., stratum corneum) and do not penetrate into the interior of the skin.

In other words, even if a biofilm formed on the surface of the human body or inside the human body is irradiated with far ultraviolet light of 190 to 240 nm in wavelength to decompose the biofilm, and the far ultraviolet light reaches the human body tissue (e.g., skin surface) after the biofilm is decomposed, the far ultraviolet light does not penetrate into the interior of the human body tissue. Therefore, it is safe for humans.

In addition, if ultraviolet rays having wavelengths of less than 190 nm are emitted into an atmosphere containing oxygen, oxygen molecules are photolyzed, yielding oxygen atoms. Then, a relatively high concentration of ozone is produced by the bond reaction between oxygen molecules and oxygen atoms. However, far ultraviolet rays in the wavelength range of 190 nm to 240 nm do not contribute to ozone formation, and thus are safe for humans and animals in terms of ozone formation.

Therefore, the decomposition and removal of biofilms and inactivation of bacteria and other organisms in biofilms by irradiation with far ultraviolet rays in the wavelength range of 190 nm to 240 nm are suitable for biofilms formed in the human body (human body surface and/or inside the human body).

Biofilms formed in the human body include, for example, biofilms formed on the surface of the human oral cavity and biofilms formed in the digestive organs (e.g., the inner lining of a large intestine). By irradiating the biofilms formed in the human body with the far ultraviolet light, the biofilms can be properly decomposed while preventing adverse effects on the human body, and the progression of periodontal disease and the occurrence of colorectal cancer can be reduced.

The wavelength range that is safe for humans and animals is preferably from 200 nm to 237 nm, more preferably from 200 nm to 235 nm, and even more preferably from 200 nm to 230 nm.

Furthermore, in order to more effectively inactivate bacteria and other organisms in biofilms, far ultraviolet irradiation may be combined with drug administration.

As mentioned above, the cell membrane of bacterial cells is damaged by far ultraviolet irradiation. Therefore, drugs (drug solutions) can easily penetrate into the interior of the bacteria after far ultraviolet irradiation. Even if some bacteria are not completely killed by far ultraviolet irradiation, by administering a bactericidal agent to bacteria after the far ultraviolet irradiation, the agent can easily penetrate into the bacteria and completely sterilize them.

In order to prevent the drug from being decomposed by the far ultraviolet light, it is preferable to stop the far ultraviolet light irradiation before administering the drug.

As described above, the ultraviolet irradiation device according to the embodiment can easily decompose biofilms by irradiating the biofilms with far ultraviolet light in a wavelength range of 190 nm and 240 nm.

The ultraviolet irradiation device according to the embodiment includes a light source part having a light source that emits far ultraviolet light in a wavelength range of 190 nm to 240 nm, and a supporting part that supports the light source part so that the far ultraviolet light emitted from the light source can be applied onto a biofilm.

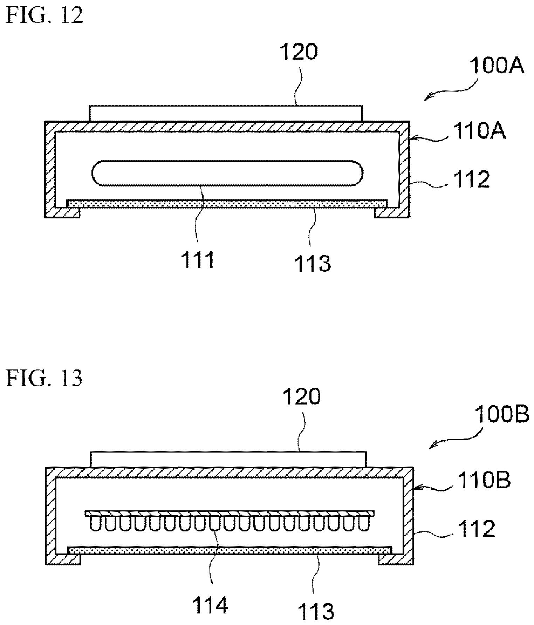

FIG. 12 is a schematic diagram showing an example of an ultraviolet irradiation device 100A according to the embodiment.

The ultraviolet irradiation device 100A shown in FIG. 12 is a device for irradiating a relatively large area with far ultraviolet light. The ultraviolet irradiation device 100A has a light source part 110A and a supporting part 120 that supports the light source part 110A.

The light source part 110A has a light source 111, an enclosure 112 that houses the light source 111, and a light radiation window 113 provided in the enclosure 112 for radiating far ultraviolet light. The light source 111 can be, for example, a KrCI excimer lamp that emits far ultraviolet light with a central wavelength of 222 nm or a KrBr excimer lamp that emits far ultraviolet light with a central wavelength of 207 nm.

The supporting part 120 supports the light source part 110A to direct the far ultraviolet light emitted from the light source (excimer lamp) 111 at the biofilm. The supporting part 120 is attached, for example, to a structure or the like provided in the vicinity of an area in which a biofilm exists (or can exist) to support the light source part 110A.

In FIG. 12, illustration of a power supply unit for supplying power to the light source 111 and a control unit for controlling the operation of the light source 111 is omitted.

The light radiation window 113 may be an ultraviolet transmissive member made of quartz glass, for example.

The spectrum of the far ultraviolet radiation emitted from the light source 111 has a certain range having a central wavelength and edges. The light source 111 may emit ultraviolet components with wavelengths near one of the edges of the range that are 240 nm or longer and are harmful to the human body. Accordingly, in a case in which the ultraviolet light from the light source part 110A is directed at a human (for example, the target to be decomposed is a biofilm formed in the human body), it is preferable that the light radiation window 113 be provided with an optical filter that transmits far ultraviolet light in the wavelength range of 190 nm and 240 nm emitted from the light source 111 and blocks or minimizes the transmission of other ultraviolet rays (e.g., UV-C waves having longer wavelengths than 240 nm).

However, if the target to be decomposed is not a biofilm formed in the human body, and is a biofilm formed, for example, in a drain of a kitchen sink, the ultraviolet irradiation device 100A does not necessarily need to be provided with the above-mentioned optical filter.

The ultraviolet light irradiation device 100A shown in FIG. 12 has an excimer lamp as a far ultraviolet light source, but may have one or more LEDs as the far ultraviolet light source.

FIG. 13 is a schematic diagram showing another example of an ultraviolet irradiation device 100B that has a light source part 110B having a light source 114 formed by LEDs as a far ultraviolet light source. The configuration other than the light source 110B may be the same as that of the ultraviolet irradiation device 100A shown in FIG. 12.

As the light source 114, any of aluminum gallium nitride (AlGaN)-based LEDs, aluminum nitride (AIN)-based LEDs, and magnesium zinc oxide (MgZnO)-based LEDs may be adopted.

In the ultraviolet irradiation device 100B shown in FIG. 13, if the target to be decomposed is a biofilm formed in the human body and if the light emitted from the light source 114 contains ultraviolet light components of which the wavelength is different from the range of 190 nm to 240 nm, it is preferable that the light radiation window 113 be provided with an optical filter that transmits far ultraviolet light in the wavelength range of 190 nm and 240 nm emitted from the light source 114 and blocks or minimizes the transmission of other ultraviolet rays (e.g., UV-C waves having longer wavelengths than 240 nm).

Although a specific embodiment has been described, the above-described embodiment is merely an example and is not intended to limit the scope of the present invention. The device and method described herein can be embodied in forms other than those described above. Also, without departing from the scope of the present invention, omissions, substitutions, and modifications may be made to the above-described embodiment appropriately. Such omissions, substitutions, and modifications are included in the scope of the claims and their equivalents, and belong to the technical scope of the present invention.

### REFERENCE SYMBOLS

10: Ultraviolet irradiation device, 20: Microplate (Well), 21: Microplate bottom surface, 30: Biofilm, 100A-100D: Ultraviolet irradiation device, 110A, 110B: Light source part, 111: Light source (excimer lamp), 112: Enclosure, 113: Optical filter, 114: Light source (LEDs), 120: Supporting part, 130: Light source, 131: LED, 132: Optical filter, 141: Body, 142: Light guide, 143: Light emitting end, 150: Insertion portion, 151: Illumination light introduction portion, 152: Observation light introduction portion, 161: Far ultraviolet light introduction portion

## Claims

1. An ultraviolet irradiation device comprising:
a light source part having a light source that emits ultraviolet light in a wavelength range of 190 nm to 240 nm for irradiating a biofilm with the ultraviolet light to decompose the biofilm.

2. The ultraviolet irradiation device according to claim 1, wherein irradiating the biofilm with the ultraviolet light causes inactivating bacteria that have formed the biofilm, and microorganisms and/or viruses contained in the biofilm.

3. The ultraviolet irradiation device according to claim 1, wherein the ultraviolet irradiation device irradiates the biofilm formed on either a surface of an oral cavity or an inner lining of a large intestine of a human with the ultraviolet light.

4. The ultraviolet irradiation device according to claim 3, wherein the light source part further has an optical filter that transmits ultraviolet light in a wavelength range of 190 nm and 240 nm and blocks transmission of UV-C waves having longer wavelengths than 240 nm, and wherein the ultraviolet irradiation device radiates the ultraviolet light in the wavelength range of 190 nm to 240 nm that passes through the optical filter among the ultraviolet light emitted from the light source.

5. A method of ultraviolet irradiation comprising:
a step of emitting ultraviolet light in a wavelength range of 190 nm to 240 nm from a light source; and
a step of irradiating a biofilm formed on either a surface of an oral cavity or an inner lining of a large intestine of a human with the ultraviolet light emitted from the light source to decompose the biofilm.

6. The method of ultraviolet irradiation according to claim 5, further comprising
a step of irradiating the biofilm with the ultraviolet light emitted from the light source to inactivate bacteria that have formed the biofilm, and microorganisms and/or viruses contained in the biofilm.

7. The method of ultraviolet irradiation according to claim 5, further comprising
a step of stopping emission of the ultraviolet light after emitting the ultraviolet light; and
a step of administering an agent to the biofilm.
